# EUROPÄISCHE PATENTSCHRIFT

(11) **EP 1 867 284 B1**
(45) Veröffentlichungstag und Bekanntmachung des Hinweises auf die Patenterteilung: **12.11.2008**
(21) Anmeldenummer: 06011730.6
(22) Anmeldetag: 07.06.2006
(51) Int. Cl.: A61B 6/04

(54) **Medizinische Standpositionierungsvorrichtung und -system**
Medical positioning system
Systeme medical de positionnement

(43) Veröffentlichungstag der Anmeldung: 19.12.2007
(73) Patentinhaber: BrainLAB AG, 85551 Kirchheim/Heimstetten (DE)
(72) Erfinder: Pousset, Philipp, 85748 Garching (DE); Fleig, Oliver, 85598 Baldham (DE)
(74) Vertreter: Schwabe - Sandmair - Marx

(56) Entgegenhaltungen:
- EP-A- 0 119 660
- WO-A-99/18855
- US-A- 3 256 611
- US-A- 3 524 057
- US-A- 3 700 894
- US-A- 4 694 478

## Beschreibung

Die Erfindung betrifft eine medizintechnische Standpositionierungsvorrichtung sowie ein medizintechnisches Standpositionierungssystem.

Solche Standpositionierungsvorrichtungen werden verwendet, um in vorgegebenen Stellungen Röntgenbilder eines stehenden Patienten aufzunehmen. Eine solche Vorrichtung ist aus dem Dokument US 4694478 bekannt. Für einige Anwendungen wird es dabei notwendig, beispielsweise zwei Patientenaufnahmen für den Hüftbereich aus unterschiedlichen Winkeln aufzunehmen, um die Bilder dann später zum Beispiel an einem Computerbildschirm auszuwerten und alle hierfür notwendigen Informationen zur Verfügung zu haben. Bei standardisierten Auswertungsverfahren ist es schon möglich, mit Hilfe dieser Bildinformationen eine grobe Vorab-Behandlungsplanung durchzuführen, also beispielsweise schon im Computersystem hinterlegte Implantate grob in das Bildmaterial einzuplanen.

Um solche Vorab-Planungsschritte durchzuführen, sollte aber schon gewährleistet sein, dass der Patient sich bei den Aufnahmen relativ genau in der gewünschten Aufnahmerichtung befindet, also beispielsweise in einer Aufnahmerichtung direkt von vorne (Anterior-Posterior) und zusätzlich in einer zweiten Aufnahme zum Beispiel in einem Winkel von 60° zum Röntgenfilm.

Es sind bisher verschiedene Versuche gemacht worden, diese vorgegebenen Patientenpositionierungen zu erreichen. Beispielsweise wurden vor dem Röntgenfilm Folien auf den Boden gelegt, die mehrere Fußstellungen aufzeigten. Der Patient wurde dann angewiesen, sich entsprechend der gewünschten Position auf den Fußabbildungen aufzustellen. Solche Folien haben den Nachteil, dass sie leicht verrutschen und dann jedes Mal wieder ausgerichtet werden müssen, beispielsweise anhand einer am Boden aufgezeichneten Linie. Wenn eine solche Neuausrichtung nicht erfolgt, kann die Patientenstellung im Röntgenbild leicht falsch sein. Ein weiterer Ansatz bestand darin, an dem Patienten ein auf den Bildern abbildbares Kalibrierungsobjekt anzubringen. Die hier verwendete Technik ist aufwändig und es besteht die Möglichkeit, dass das Kalibrierungsobjekt sich relativ zum Patienten bewegt, während dieser sich neu positioniert. Dadurch würde die Positionsbestimmung ungenau. Aus der US 3,700,894 ist eine Patientenpositionierungsvorrichtung bekannt, die an drehbaren Stangen angebrachte Fußanschläge sowie Kopfhalterungen umfasst. Die Vorrichtung ist aber wiederum sehr aufwändig gestaltet und muss permanent an ihrem Ort verbleiben. Dokument EP 0 119 660 offenbart eine Vorrichtung mit einer drehbaren Scheibe.

Es ist die Aufgabe der vorliegenden Erfindung, eine medizintechnische Standpositionierung zu ermöglichen, welche eine genaue Positionierung des Patienten bei geringem Aufwand möglich macht. Mindestens einige der oben genannten Nachteile des Standes der Technik sollen dabei überwunden werden.

Diese Aufgabe wird durch eine medizintechnische Standpositionierungsvorrichtung gemäß dem Anspruch 1 gelöst. Die Unteransprüche definieren bevorzugte Ausführungsformen der Erfindung.

Eine medizintechnische Standpositionierungsvorrichtung gemäß der vorliegenden Erfindung weist ein Fußaufsatz-Flächengebilde auf, das Fußpositionierungseinrichtungen umfasst. Außerdem hat das Fußaufsatz-Flächengebilde an seinen Außenkanten Anschlag- oder Eingriffsmittel, die in einem vordefinierten Positionsverhältnis zu den Fußpositionierungseinrichtungen stehen.

Mit anderen Worten wird die erfindungsgemäße Standpositionierungsvorrichtung so ausgebildet, dass ihr Außenumfang schon so ausgestaltet ist, dass er als Ausrichtungshilfe für die Vorrichtung insgesamt dienen kann. Die erfindungsgemäße Vorrichtung umfasst also gleichzeitig die Anzeige für den Patienten, die ihm angibt, wo er seine Füße positionieren soll, und die Ausrichtungshilfe für die Vorrichtung selbst, nämlich an dem äußeren Umfang des Fußaufsatz-Flächengebildes. Weil die Außenkanten Anschläge oder Eingriffe aufweisen, kann dabei eine korrekte Ausrichtung der erfindungsgemäßen Vorrichtung und damit auch eine korrekte Ausrichtung des Patienten in sehr einfacher Weise sichergestellt werden.

Gegenüber den Methoden, die Kalibrierungsobjekte verwenden, hat die vorliegende Erfindung den Vorteil, dass Bildverarbeitungsfehler, die aufgrund von verschobenen Kalibrierungsobjekten zustande kommen, nicht auftreten; außerdem entfällt die Notwendigkeit, eine entsprechende Bildverarbeitung überhaupt durchzuführen. Weil die erfindungsgemäße Vorrichtung sehr unkompliziert und mit wenigen Bauteilen zur Verfügung gestellt werden kann, hebt sie sich auch vorteilhaft von komplizierten, technisch aufwändigen und permanent am Untersuchungsort verbleibenden Vorrichtungen ab, wie sie in der US 3,700,894 beschrieben werden. Die erfindungsgemäße Vorrichtung definiert implizit durch ihre Ausgestaltung bzw. Konstruktion die Winkelausrichtung der Fußpositionierungseinrichtungen, so dass auch nicht unbeabsichtigt ein falscher Winkel gewählt werden kann. Weil die Außenkanten der erfindungsgemäßen Vorrichtung ihre Ausrichtung definieren, muss sie lediglich an ein entsprechend ausgerichtetes Gegenstück angelegt werden, beispielsweise an die Wand, an der auch der Röntgenfilmhaltern angeordnet ist. Ihre Verwendung erschließt sich von selbst und es müssen keine zusätzlichen Einstellungen getroffen werden. Eine erfindungsgemäße Standpositionierungsvorrichtungen kann in einer leicht bauenden Ausführung und mit gut handhabbaren Abmessungen zur Verfügung gestellt werden; es ist keine komplizierte Installation notwendig, und sie kann nach der Verwendung leicht aus dem Arbeitsbereich entfernt und platzsparend verstaut werden.

Gemäß einer Ausführungsform der vorliegenden Erfindung stehen mehrere, insbesondere mindestens zwei Anschlag- oder Eingriffsmittel in unterschiedlichen vordefinierten Positionsverhältnissen zu den Fußpositionierungseinrichtungen, insbesondere in unterschiedlicher Winkelanordnung in Bezug auf die Fußpositionierungseinrichtung.

Das Fußaufsatz-Flächengebilde kann erfindungsgemäß eine starre Platte aufweisen, und die Anschlag- oder Eingriffsmittel können durch die Außenkanten der Platte gebildet werden oder an den Außenkanten vorgesehen sein. Ferner besteht die Möglichkeit, dass die Anschlag- oder Eingriffsmittel durch mindestens zwei Außenkanten gebildet werden, die in einem vordefinierten Winkel zueinander angeordnet sind, insbesondere in einem Außenwinkel von 200° bis 280°, speziell in einem Außenwinkel von 240°. In letzterem Fall wäre der Patient dann um 60° zwischen zwei Aufnahmen verdreht, wenn die erfindungsgemäße Vorrichtung durch Positionierung an zwei verschiedenen Außenkanten verwendet wird.

Die Außenkanten können unterschiedliche Ausgestaltungen annehmen. Sie können geradlinige, durchgehende Anschlagkanten sein oder auch einhängbare Hintergriffe, insbesondere Einhängevorsprünge bzw. Einhängenuten entlang zumindest eines Teils ihrer Länge aufweisen. Eine andere Möglichkeit der Ausgestaltung besteht darin, an den Außenkanten hinterschnittene Ausnehmungen anzuordnen. Natürlich können auch hinterschnittene Vorsprünge angeordnet sein, je nachdem wie das Gegenstück zur Positionierung der erfindungsgemäßen Vorrichtung aussieht.

Weitere Ausgestaltungen betreffen die Fußpositionierungseinrichtungen. Sie können beispielsweise für mindestens einen Fuß eine Fersenausnehmung und pro Fuß insbesondere nur einen wirksam seitlich positionierenden Fußanschlag aufweisen. Durch eine solche Fersenausnehmung wird sichergestellt, dass der Patientenfuß nicht mehr verrutschen kann, und es genügt ein einziger seitlicher, zum Beispiel medialer Fußanschlag, um eine definierte Fußausrichtung herzustellen.

Der Fußanschlag kann dabei ein nach oben von dem Fußaufsatz-Flächengebilde vorstehende Anschlagkante aufweisen.

Die Erfindung betrifft gemäß einem weiteren Aspekt ein System aus einer medizintechnischen Standpositionierungsvorrichtung, wie sie oben in verschiedenen Ausführungen erläutert wurde, und einem Positionierungs-Gegenelement, welches ein zum Anschlag- oder Eingriffsmittel der Standpositionierungsvorrichtung komplementäres Anschlag- oder Eingriffsmittel aufweist. Natürlich kann die erfindungsgemäße Standpositionierungsvorrichtung beispielsweise einfach mit einer ihrer Kanten an einer Wand angelehnt werden, und sofort stünden die Fußpositionierungseinrichtungen in der gewünschten Stellung. Bei manchen Anwendungen könnte es aber von Vorteil sein, wenn ein Gegenelement zur Verfügung gestellt wird, beispielsweise wenn der Röntgenfilmhalter nicht direkt an einer Wand angebracht ist. Wegen der einfachen Ausgestaltung der erfindungsgemäßen Standpositionierungsvorrichtung kann auch ein ebenso einfach ausgestaltetes Gegenelement bereitgestellt werden, das beispielsweise eine geradlinige, durchgehende Anschlagkante, einhängbare Hintergriffe, insbesondere Einhängevorsprünge bzw. Einhängenuten entlang zumindest eines Teils seiner Länge, hinterschnittene Vorsprünge oder hinterschnittene Ausnehmungen bzw. eine Kombination solcher Vorrichtungen umfasst. Bei einer Ausführungsvariante für das erfindungsgemäße System werden Boden- oder Wandbefestigungseinrichtungen für das Positionierungs-Gegenelement bereitgestellt, insbesondere Reibmittel, Haftmittel, Steckmittel oder mechanische Feststellmittel, wie Verschraubungen. Es können auch lösbare Befestigungseinrichtungen verwendet werden, um das Gegenelement ebenso wie die erfindungsgemäße Standpositionierungsvorrichtung nach dem Gebrauch wieder entfernen und verstauen zu können. Ein Beispiel hierfür wären Befestigungseinrichtungen wie Saugnäpfe, Klettverschlüsse oder lösbare Haftklebeschichten.

Die Erfindung wird im Weiteren anhand mehrerer Ausführungsformen und unter Bezugnahme auf die beiliegenden Zeichnungen näher erläutert. Sie kann alle hierin beschriebenen Merkmale in jedweder sinnvollen Kombination oder einzeln aufweisen. In den beiliegenden Zeichnungen zeigen:
- Figur 1: eine Aufsicht sowie einen Schnitt A-A für eine erfindungsgemäße Standpositionierungsvorrichtung;
- Figur 2: Ansichten entsprechend der Figur 1 für eine erfindungsgemäße Standpositionierungseinrichtung mit hinterschnittenen Ausnehmungen an den Positionierungskanten;
- Figuren 3A und 3B: ein erfindungsgemäßes Positionierungssystem mit einer Art Nut-Feder-Kantenpositionierung; und
- Figuren 4A und 4B: ein erfindungsgemäßes Positionierungssystem mit einer puzzleartigen Verbindung zwischen Standpositionierungsvorrichtung und Positionierungs-Gegenelement.

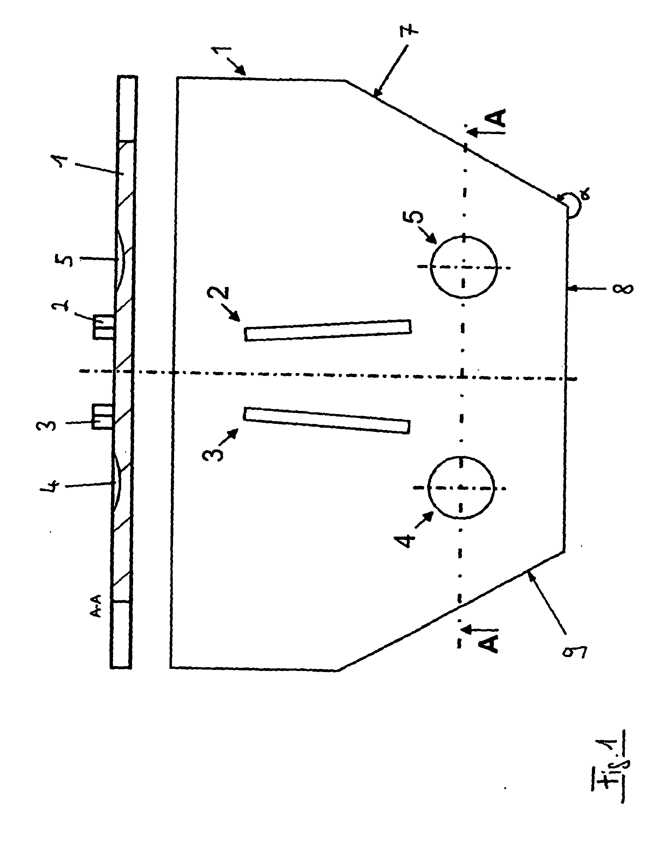

Eine einfache Ausführung der erfindungsgemäßen Standpositionierungsvorrichtung ist in der Figur 1 dargestellt, wobei die obere Darstellung den Schnitt A-A in der unteren Draufsicht zeigt. Die Positionierungseinrichtung besteht im Wesentlichen aus einer Platte, die das Bezugszeichen 1 trägt. Die Platte 1 weist eine Dicke auf, die so gestaltet ist, dass etwa kreisförmige Vertiefungen 4, 5 in sie eingebracht werden können, in welche ein Patient sich mit seinen Fersen hineinstellen kann. Typischerweise ist die Platte dicker als 1 cm, und sie kann bis zu 3 oder 5 cm dick sein. Vom Material her ist darauf zu achten, dass die Platte handhabbar sein soll, also ist ein relativ leichtes Material auszuwählen, beispielsweise Holz oder Kunststoff. Wie schon angedeutet, weist die Platte auf ihrer oberen Oberfläche zwei Fersenvertiefungen 4, 5 auf; außerdem sind zwei mediale Fußanschläge 2 und 3 vorgesehen. Ein Patient kann sich jeweils mit einer Ferse in eine der Vertiefungen 4, 5 stellen und dann seine Fußinnenseite an den jeweiligen Fußanschlag 3, 2 anlehnen, und er steht dann definiert in einer reproduzierbaren Position.

Die Platte hat einen erfindungsgemäß ausgebildeten Außenumfang, und dieser wird durch die Außenkanten 7, 8 und 9 gebildet. Die Kanten 7, 8, 9 sind im vorliegenden Fall Anschlagkanten, d.h. die Platte 1 wird mit dieser Kante beispielsweise an einer Wand anstoßend positioniert. Wenn die Platte mit der Kante 8 an einer Wand anliegend positioniert wird, an der ein Röntgenfilmträger angebracht ist, wird der Patient exakt parallel zur Wand stehen und es kann eine Anterior-Posterior-Aufnahme erstellt werden. Die beiden Kanten 7 und 9 liegen in genau definierten Winkelverhältnissen zur Kante 8, und in der Figur 1 ist beispielsweise der Winkel α angegeben, ein Außenwinkel von 240°. Wenn die Platte nach der ersten Anterior-Posterior-Aufnahme verdreht und beispielsweise mit der Kante 7 an derselben Wand wie vorher beschrieben angelegt wird, kann eine Aufnahme gemacht werden, bei der der Patient um 60° verdreht schräg von vorne aufgenommen wird.

Mit dieser Ausführungsform ist es also besonders einfach, Röntgenaufnahmen in exakt definierten Positionen des Patienten zu erstellen, und zwar einfach indem die Platte zweimal mit unterschiedlichen Kanten an die Wand angelegt wird. Die Platte kann danach wieder verstaut werden und nimmt im Behandlungsbereich keinen Platz mehr weg.

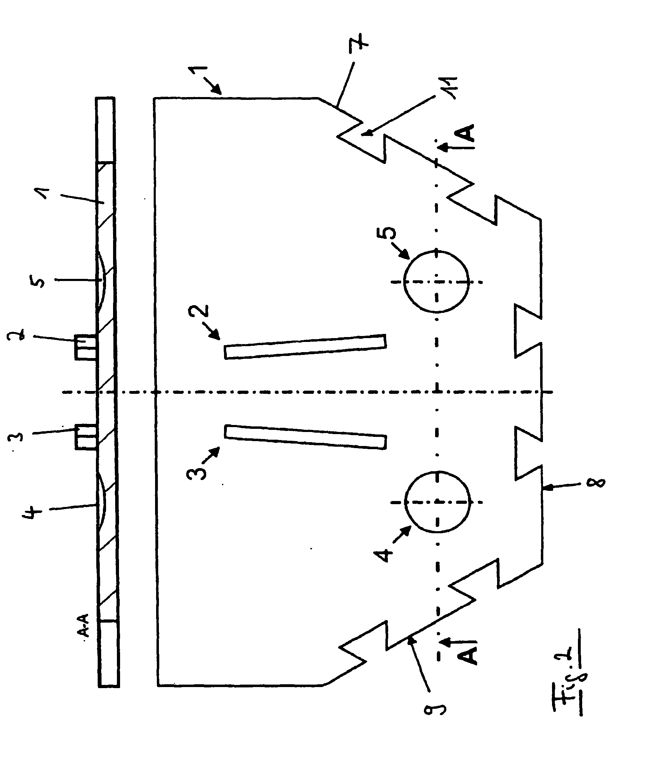

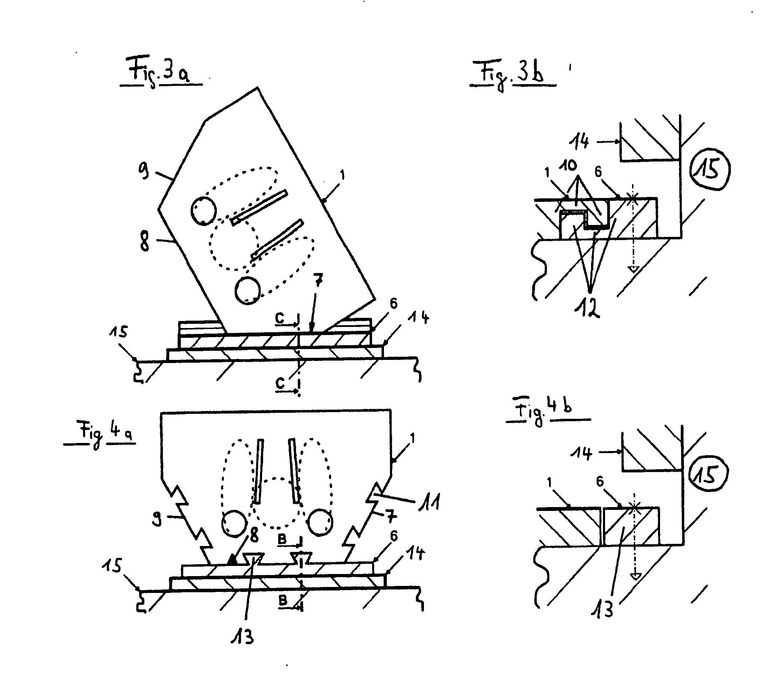

In der Figur 2 ist in einer Darstellung, die derjenigen aus Figur 1 entspricht, eine weitere Ausführungsform einer erfindungsgemäßen Positionierungsplatte aufgezeigt. Im Unterschied zu den glatten, geradlinigen Anschlagkanten der Ausführungsform nach Figur 1 weist die Platte in Figur 2 an den Kanten 7, 8, 9 Ausnehmungen auf, von denen eine mit dem Bezugszeichen 11 gekennzeichnet worden ist. Diese Aufnehmungen weiten sich rautenförmig von der Kante aus auf und bilden damit Hinterschneidungen, mit denen die Platte 1 sehr exakt an einem Positionierungs-Gegenelement verankert werden kann. Um dies näher zu erläutern, wird nunmehr zunächst auf die Figuren 4A und 4B Bezug genommen, in denen eine Platte gemäß der Figur 2 sowie ein Positionierungs-Gegenelement dargestellt ist, wobei letzteres das Bezugszeichen 6 trägt. Das Gegenelement 6 ist im vorliegenden Fall eine im Boden verschraubte Leiste, die nach vorne vorstehende, sich rautenförmig aufweitende Vorsprünge 13 umfasst. Die Vorsprünge 13 passen in die Ausnehmungen 11; diese beiden Elemente sind komplementär. Wenn nun, wie in Figur 4A gezeigt ist, die Platte mit den Ausnehmungen 11 an der Kante 8 auf die Vorsprünge 13 aufgesetzt wird, so dass diese sich in die Ausnehmungen 11 einfügen, wird die Platte 1 durch das Gegenelement 6 unverrückbar positioniert., und zwar exakt in dem Winkel, in dem eine Röntgenaufnahme durchgeführt werden soll. Dies gilt natürlich auch für die anderen Kanten mit Ausnehmungen 11, wenn andere Winkel zu wählen sind.

Die Figur 4B zeigt noch, dass das Gegenelement 6 im Bereich der Wand 15 etwa unterhalb des Röntgenfilmhalters 14 angeordnet ist; die Figur 4B ist ein Schnitt an der Ebene, die in Figur 4A mit B-B bezeichnet ist.

Eine andere Art der Positionierung einer erfindungsgemäßen Positionierungsplatte 1 ist in den Figuren 3A und 3B gezeigt, wobei die Figur 3B einen Schnitt an der Ebene darstellt, die in der Figur 3A mit C-C aufgezeigt ist. Die Platte 1 in Figur 3A umfasst an ihren Außenkanten 7, 8 und 9 eine nach unten offene Einhängenut in dem Randbereich, der in der Figur 3B mit dem Bezugszeichen 10 angedeutet ist. Eine komplementäre, nach oben offene Nut ist am Gegenelement 6 angebracht, und der entsprechende Bereich ist in Figur 3B mit dem Bezugszeichen 12 gekennzeichnet. Die Platte 1 aus der Figur 3A kann mit dem Randbereich 10 in den Randbereich 12 des Gegenelements 6 von oben eingehängt werden, und sie ist dann wie auf einer Schiene in ihrer jeweiligen Position angeordnet, um jeweils bei der Benutzung der Außenkanten 7, 8 oder 9 die vorgesehenen und vordefinierten Winkelstellungen für den Patienten bereitzustellen. Auch hier ist wieder in der Figur 3B das Gesamt-Positionsverhältnis zwischen Platte 1, Gegenelement 6 und der Wand 15 mit dem Röntgenfilmhalter 14 zu sehen.

Natürlich kann eine erfindungsgemäße Standpositionierungsvorrichtung auch im System mit dem Gegenelement noch verschiedene andere Ausgestaltungen annehmen, beispielsweise können die puzzleartigen Ausnehmungen und Vorsprünge 11 und 12 durch andere, entsprechende Varianten ersetzt werden, unter anderem auch durch Ausnehmungen und Vorsprünge ohne Hinterschneidungen. Außerdem könnte beispielsweise zusammen mit der Platte 1 noch ein zusätzliches, hier nicht dargestelltes, Abstandselement bereitgestellt werden, das einfach die Form eines flachen Balkens hat, um die Platte nach Figur 1 entsprechend weiter von einer Wand, aber trotzdem noch exakt winkelmäßig zu positionieren. Auch solche Abstandsstücke könnten mit Ausnehmungen und Vertiefungen bzw. mit Einhängevorrichtungen versehen werden, um dann zu Ausführungsformen gemäß den Figuren 2 oder 4A bzw. 4B zu passen.

## Patentansprüche

1. Medizintechnische Standpositionierungsvorrichtung mit einem Fußaufsatz-Flächengebilde (1), das Fußpositionierungseinrichtungen (2, 3, 4, 5) umfasst, wobei das Fußaufsatz-Flächengebilde (1) an seinen Außenkanten Anschlag- oder Eingriffsmittel (7, 8, 9, 10, 11) umfasst, die in einem vordefinierten Positionsverhältnis zu den Fußpositionierungseinrichtungen (2, 3, 4, 5) stehen, **dadurch gekennzeichnet, dass** die Anschlag- oder Eingriffsmittel (7, 8, 9, 10, 11) durch mindestens zwei Außenkanten (7, 8, 9) des Fußaufsatz-Flächengebildes (1) gebildet werden oder an mindestens zwei Außenkanten vorgesehen sind.

2. Standpositionierungsvorrichtung nach Anspruch 1, **dadurch gekennzeichnet, dass** mehrere, insbesondere mindestens zwei Anschlag- oder Eingriffsmittel (7, 8, 9, 10, 11) in unterschiedlichen vordefinierten Positionsverhältnissen zu den Fußpositionierungseinrichtungen (2, 3, 4, 5) stehen, insbesondere in unterschiedlicher Winkelanordnung in Bezug auf die Fußpositionierungseinrichtungen (2, 3, 4, 5).

3. Standpositionierungsvorrichtung nach Anspruch 1 oder 2, **dadurch gekennzeichnet, dass** das Fußaufsatz-Flächengebilde (1) eine starre Platte aufweist.

4. Standpositionierungsvorrichtung nach einem der Ansprüche 1 bis 3, **dadurch gekennzeichnet, dass** die Außenkanten in einem vordefinierten Winkel zueinander angeordnet sind, insbesondere in einem Außenwinkel von 200° bis 280°, speziell in einem Außenwinkel von 240°.

5. Standpositionierungsvorrichtung nach einem der Ansprüche 1 bis 4, **dadurch gekennzeichnet, dass** die Außenkanten geradlinige, durchgehende Anschlagkanten sind.

6. Standpositionierungsvorrichtung nach einem der Ansprüche 1 bis 4, **dadurch gekennzeichnet, dass** die Außenkanten einhängbare Hintergriffe (10), insbesondere Einhängevorsprünge bzw. Einhängenuten entlang zumindest eines Teils ihrer Länge aufweisen.

7. Standpositionierungsvorrichtung nach einem der Ansprüche 1 bis 4, **dadurch gekennzeichnet, dass** an den Außenkanten hinterschnittene Ausnehmungen (11) oder Vorsprünge angeordnet sind.

8. Standpositionierungsvorrichtung nach einem der Ansprüche 1 bis 7, **dadurch gekennzeichnet, dass** die Fußpositionierungseinrichtungen (2, 3, 4, 5) für mindestens einen Fuß eine Fersenausnehmung (4, 5), und insbesondere nur einen wirksam seitlich positionierenden Fußanschlag (2, 3) aufweisen.

9. Standpositionierungsvorrichtung nach Anspruch 8, **dadurch gekennzeichnet, dass** der Fußanschlag eine nach oben von dem Fußaufsatz-Flächengebilde (1) vorstehende Anschlagkante aufweist.

10. System aus einer medizintechnischen Standpositionierungsvorrichtung nach einem der Ansprüche 1 bis 9 und einem Positionierungs-Gegenelement (6), welches ein zum Anschlag- oder Eingriffsmittel (7, 8, 9, 10, 11) der Standpositionierungsvorrichtung komplementäres Anschlag- oder Eingriffsmittel (12, 13) aufweist.

11. System nach Anspruch 10, **dadurch gekennzeichnet, dass** das Positionierungs-Gegenelement (6)
- eine geradlinige, durchgehende Anschlagkante;
- einhängbare Hintergriffe (12), insbesondere Einhängevorsprünge bzw. Einhängenuten entlang zumindest eines Teils seiner Länge;
- hinterschnittene Vorsprünge (11) oder Ausnehmungen;
oder eine Kombination solcher Vorrichtungen umfasst.

12. System nach Anspruch 10 oder 11, **dadurch gekennzeichnet, dass** es Boden- oder Wandbefestigungseinrichtungen für das Positionierungs-Gegenelement (6) umfasst, insbesondere Reibmittel, Haftmittel, Steckmittel oder mechanische Feststellmittel wie Verschraubungen.

## Claims

1. A medical upright positioning device, comprising a foot attachment planar structure (1) comprising foot positioning means (2, 3, 4, 5), wherein the outer edges of the foot attachment planar structure (1) comprise abutting or engaging means (7, 8, 9, 10, 11) which are in a predefined positional relationship to the foot positioning means (2, 3, 4, 5), **characterised in that** the abutting or engaging means (7, 8, 9, 10, 11) are formed by at least two outer edges (7, 8, 9) of the foot attachment planar structure (1) or are provided on at least two outer edges.

2. The upright positioning device according to claim 1, **characterised in that** multiple abutting or engaging means (7, 8, 9, 10, 11), in particular at least two, are in different predefined positional relationships to the foot positioning means (2, 3, 4, 5), in particular in different angular arrangements in relation to the foot positioning means (2, 3, 4, 5).

3. The upright positioning device according to claim 1 or 2, **characterised in that** the foot attachment planar structure (1) comprises a rigid plate.

4. The upright positioning device according to any one of claims 1 to 3, **characterised in that** the outer edges are arranged at a predefined angle to each other, in particular at an external angle of 200° to 280°, specifically at an external angle of 240°.

5. The upright positioning device according to any one of claims 1 to 4, **characterised in that** the outer edges are linear, continuous abutting edges.

6. The upright positioning device according to any one of claims 1 to 4, **characterised in that** the outer edges comprise hooking engagements (10), in particular hooking protrusions and/or hooking grooves along at least a part of their length.

7. The upright positioning device according to any one of claims 1 to 4, **characterised in that** undercut cavities (11) or protrusions are arranged on the outer edges.

8. The upright positioning device according to any one of claims 1 to 7, **characterised in that** the foot positioning means (2, 3, 4, 5) comprise a heel cavity (4, 5) for at least one foot and in particular only one effectively laterally positioning foot stopper (2, 3) per foot.

9. The upright positioning device according to claim 8, **characterised in that** the foot stopper comprises an abutting edge which protrudes upwards from the foot attachment planar structure (1).

10. A system consisting of a medical upright positioning device according to any one of claims 1 to 9 and a positioning counter element (6) which comprises an abutting or engaging means (12, 13) which is complementary to the abutting or engaging means (7, 8, 9, 10, 11) of the upright positioning device.

11. The system according to claim 10, **characterised in that** the positioning counter element (6) comprises:
- a linear, continuous abutting edge;
- hooking engagements (12), in particular hooking protrusions and/or hooking grooves along at least a part of its length;
- undercut protrusions (11) or undercut cavities;
or a combination of such devices.

12. The system according to claim 10 or 11, **characterised in that** it comprises floor fastening means or wall fastening means for the positioning counter element (6), in particular friction means, adhesion means, locking means or mechanical fixing means such as screw connections.

## Revendications

1. Dispositif médical de positionnement debout avec une structure plate repose-pieds (1) qui comprend des dispositifs de positionnement des pieds (2, 3, 4, 5), la structure plate repose-pieds (1) comprenant sur ses bords extérieurs des moyens de butée ou d'engagement (7, 8, 9, 10, 11) qui sont dans un rapport de position prédéfini avec les dispositifs de positionnement des pieds (2, 3, 4, 5), **caractérisé en ce que** les moyens de butée ou d'engagement (7, 8, 9, 10, 11) sont formés par au moins deux bords extérieurs (7, 8, 9) de la structure plate repose-pieds (1) ou sont prévus sur au moins deux bords extérieurs.

2. Dispositif de positionnement debout selon la revendication 1, **caractérisé en ce que** plusieurs, en particulier au moins deux moyens de butée ou d'engagement (7, 8, 9, 10, 11) sont dans des rapports de position prédéfinis différents avec les dispositifs de positionnement des pieds (2, 3, 4, 5), en particulier dans une disposition angulaire différente par rapport aux dispositifs de positionnement des pieds (2, 3, 4, 5).

3. Dispositif de positionnement debout selon la revendication 1 ou 2, **caractérisé en ce que** la structure plate repose-pieds (1) comporte une plaque rigide.

4. Dispositif de positionnement debout selon l'une quelconque des revendications 1 à 3, **caractérisé en ce que** les bords extérieurs sont disposés l'un par rapport à l'autre suivant un angle prédéfini, en particulier un angle extérieur de 200° à 280°, plus spécialement suivant un angle extérieur de 240°.

5. Dispositif de positionnement debout selon l'une quelconque des revendications 1 à 4, **caractérisé en ce que** les bords extérieurs sont des bords de butée continus, rectilignes.

6. Dispositif de positionnement debout selon l'une quelconque des revendications 1 à 4, **caractérisé en ce que** les bords extérieurs comportent des prises arrière (10) à accrocher, en particulier des saillies d'accrochage ou des rainures d'accrochage le long d'au moins une partie de leur longueur.

7. Dispositif de positionnement debout selon l'une quelconque des revendications 1 à 4, **caractérisé en ce que** des évidements (11) détalonnés ou des saillies sont disposés sur les bords extérieurs.

8. Dispositif de positionnement debout selon l'une quelconque des revendications 1 à 7, **caractérisé en ce que** les dispositifs de positionnement des pieds (2, 3, 4, 5) comportent, pour au moins un pied, un évidement pour talon (4, 5) et en particulier uniquement une butée de pied (2, 3) de positionnement opérant latéralement.

9. Dispositif de positionnement debout selon la revendication 8, **caractérisé en ce que** la butée de pied présente une arête de butée faisant saillie vers le haut depuis la structure plate repose-pieds (1).

10. Système constitué d'un dispositif médical de positionnement debout selon l'une quelconque des revendications 1 à 9 et d'un contre-élément de positionnement (6) qui comporte un moyen de butée ou d'engagement (12, 13) complémentaire du moyen de butée ou d'engagement (7, 8, 9, 10, 11) du dispositif de positionnement debout.

11. Système selon la revendication 10, **caractérisé en ce que** le contre-élément de positionnement (6) comprend
- une arête de butée continue et rectiligne ;
- des prises arrière (12) pouvant être accrochées, en particulier des saillies d'accrochage ou des rainures d'accrochage le long d'au moins une partie de sa longueur ;
- des saillies détalonnées (11) ou des évidements ;
ou une combinaison de ces dispositifs.

12. Système selon la revendication 10 ou 11, **caractérisé en ce qu'**il comprend des dispositifs de fixation de fond ou de paroi pour le contre-élément de positionnement (6), en particulier des moyens à friction, des moyens adhésifs, des moyens à enfichage ou des moyens de blocage mécaniques tels que des vissages.
